# EUROPEAN PATENT APPLICATION

(11) **EP 4 151 649 A1**
(43) Date of publication of application: **22.03.2023**
(21) Application number: 21857136.2
(22) Date of filing: 24.02.2021
(51) Int. Cl.: C07K 16/24, C12N 15/63, G01N 33/53, A61K 39/395, A61P 37/00

(54) **IL-5 BINDING MOLECULE, PREPARATION METHOD THEREFOR, AND USE THEREOF**

(30) Priority: 20.08.2020 CN 202010843501
(71) Applicant: Regenecore Biotech Co., Ltd, Nanjing, Jiangsu 210000 (CN)
(72) Inventor: SU, Zhipeng, Nanjing, Jiangsu 210000 (CN); MENG, Jinguo, Nanjing, Jiangsu 210000 (CN); ZHANG, Yun, Nanjing, Jiangsu 210000 (CN); WANG, Lefei, Nanjing, Jiangsu 210000 (CN); YAO, Yao, Nanjing, Jiangsu 210000 (CN)
(74) Representative: Petraz, Gilberto Luigi
(86) International application number: PCT/CN2021/077650
(87) International publication number: WO 2022/037031

(57) **Abstract**

Disclosed are an IL-5 binding molecule, and a preparation method and use thereof. The binding molecule includes the following complementarity determining regions: an amino acid sequence of CDR1 selected from any one of sequences as set forth in SEQ ID NOs. 43-49; an amino acid sequence of CDR2 selected from any one of sequences as set forth in SEQ ID NOs. 50-56; and an amino acid sequence of CDR3 selected from any one of sequences as set forth in SEQ ID NOs. 57-62. The binding molecule is capable of specifically binding to IL-5, and effectively blocking the cell proliferation induced by IL-5, and can be used for the prevention, diagnosis, and/or treatment of IL-5-associated diseases such as autoimmune disease.

## Description

### TECHNICAL FIELD

The present disclosure relates to the field of pharmaceutical biotechnologies, and specifically, to a binding molecule for IL-5, and a preparation method and use thereof.

### BACKGROUND

Human interleukin 5, also referred to as IL-5, together with IL-13 and granulocyte macrophage-colony stimulating factor (GM-CSF) are cytokines involved in hematopoiesis and inflammation. All of the three cytokines can promote the production, function, and survival of eosinophils, and thus are capable of affecting inflammatory diseases. For example, eosinophils mainly function as an effector for asthma, atopic dermatitis, and allergic rhinitis.

IL-5, as an eosinophil-specific cytokine, has been focused on by most of researchers, with increased IL-5 mRNA and protein levels in lung tissue and bronchoalveolar lavage (BAL) from patients with symptomatic asthma. It was also observed by researchers that there is a correlation between IL-5 levels and allergen provocation and disease activity. However, it is clear that in addition to IL-5, GM-CSF and IL-3 function in eosinophil production and activation for asthma, GM-CSF and IL-3 are demonstrated to be synthesized where allergic inflammation occurs. The expression of these cytokines may help increase the total infiltrating eosinophils and the degree of eosinophil activation. It is also possible that these cytokines function in eosinophil infiltration at different stages. For recent kinetic data from patients who received antigen challenge, it is showed that the IL-5 level increased from day 2 to day 7, and the GM-CSF level peaked at day 2 and kept rising at day 16.

IL-5, GM-CSF and IL-3 stimulate eosinophils and other normal and cancer cells by binding to cell surface receptors. The cell surface receptor includes a ligand-specific α chain and a chain (βc) shared by the three receptors. Binding to the α chain of each receptor is the initial step for receptor activation. However, binding to the α chain alone is not sufficient for activation. Subsequently, the ligand recruits βc, followed by a step with two major functional consequences as follows. First, it allows the binding of IL-5, GM-CSF, and IL-3 to become essentially irreversible; and then, it results in full activation of the receptor. As the major signal transduction component of these receptors, βc leads to the activation of JAK-2, STAT-5, and other signaling molecules, and ultimately leads to excess cellular activity normally associated with IL-5, stimulation of GM-CSF and IL-3 such as eosinophils adhesion, resulting in degranulation and cytotoxicity, and prolonging cell viability.

To block or antagonize the activity of cytokines for eosinophil activation, researchers have tried three main methods. One of them adopts antibodies against the cytokines involved. For example, the antibody against IL-5 is used in an animal model with allergen-induced asthma. This method has showed a relatively long-acting effect in preventing from high response due to eosinophil influx into airway and bronchia. However, there is still a lack of high-affinity, IL-5-specific antibodies, or IL-5-targeted drugs. In view of this, the present disclosure is provided.

### SUMMARY

An objective of the present disclosure is to provide an IL-5 binding molecule, and a preparation method and use thereof.

The present disclosure is implemented as follows.

According to a first aspect, an embodiment of the present disclosure provides an IL-5 binding molecule, being capable of specifically binding to IL-5 and comprising at least one of immunoglobulin single variable domains comprising complementarity determining regions CDR1, CDR2, and CDR3.

An amino acid sequence of CDR1 is selected from any one of sequences as set forth in SEQ ID NOs. 43-49; an amino acid sequence of CDR2 is selected from any one of sequences as set forth in SEQ ID NOs. 50-56; and an amino acid sequence of CDR3 is selected from any one of sequences as set forth in SEQ ID NOs. 57-62.

According to a second aspect, an embodiment of the present disclosure provides an isolated nucleotide, encoding the IL-5 binding molecule according to the foregoing embodiment.

According to a third aspect, an embodiment of the present disclosure provides a recombinant vector, comprising the isolated nucleotide according to the foregoing embodiment.

According to a fourth aspect, an embodiment of the present disclosure provides a host cell, comprising the recombinant vector according to the foregoing embodiment.

According to a fifth aspect, an embodiment of the present disclosure provides a preparation method of an IL-5 binding molecule, comprising culturing the host cell according to the foregoing embodiment to obtain the IL-5 binding molecule.

According to a sixth aspect, an embodiment of the present disclosure provides a conjugate for binding an IL-5 protein, comprising a conjugation component and the IL-5 binding molecule according to the foregoing embodiments, wherein
the conjugation component is conjugated to the IL-5 binding molecule; and the conjugation component comprises a marker and/or compound for detection.

According to a seventh aspect, an embodiment of the present disclosure provides a kit for detecting IL-5, comprising the IL-5 binding molecule according to the foregoing embodiments.

According to an eighth aspect, an embodiment of the present disclosure provides use of the IL-5 binding molecule according to the above embodiments in preparing IL-5-targeted drugs for treatment of a disease.

The present disclosure has the following beneficial effects.

The embodiments of the present disclosure provide an IL-5 binding molecule, and a preparation method and use thereof. The binding molecule is capable of specifically binding to IL-5 and comprises at least one of immunoglobulin single variable domains comprising complementarity determining regions CDR1, CDR2, and CDR3. An amino acid sequence of CDR1 is selected from any one of sequences as set forth in SEQ ID NOs. 43-49; an amino acid sequence of CDR2 is selected from any one of sequences as set forth in SEQ ID NOs. 50-56; and an amino acid sequence of CDR3 is selected from any one of sequences as set forth in SEQ ID NOs. 57-62. The binding molecule is capable of specifically binding to IL-5, and effectively blocking the cell proliferation induced by IL-5, and can be used for the prevention, diagnosis, and/or treatment of IL-5-related diseases.

### BRIEF DESCRIPTION OF THE DRAWINGS

To describe the technical solutions of the embodiments of the present disclosure more clearly, the following briefly describes the accompanying drawings required for describing the embodiments. It should be understood that, the following accompanying drawings show only some embodiments of the present disclosure, which cannot be considered as limitation on the scope. A person of ordinary skill in the art may still derive other accompanying drawings from the accompanying drawings without creative efforts.
FIG. 1 shows gel electrophoresis results of the human recombinant IL-5 protein in Example 1;

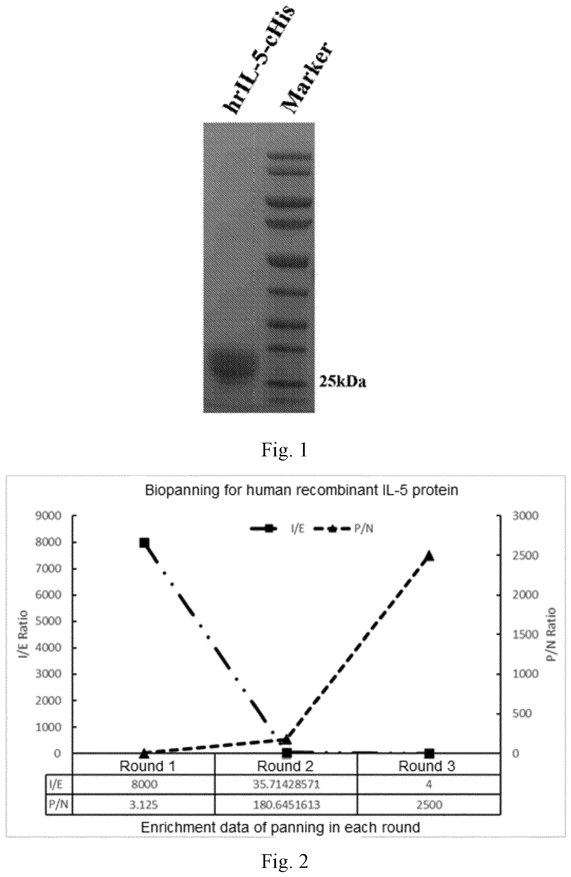

FIG. 2 shows the enrichment for the IL-5 recombinant protein after the screening of the nanobody library in Example 1; PIN = number of monoclonal bacteria grown after TG1 bacteria are infected with the phages eluted from positive wells in biopanning/number of monoclonal bacteria grown after TG1 bacteria are infected with the phages eluted from positive wells, where this parameter gradually increases during the enrichment; I/E = total amount of phages added in positive wells in each round in biopanning/total amount of phages eluted from positive wells in biopanning, where this parameter gradually approaches 1 during the enrichment;
FIG. 3 shows respective alignment results of humanized variants of antibody strains 1B3 and 2B3 in Example 2;
FIG. 4 shows the analysis results of binding of 12 strains of antibodies expressed by E. coli obtained from Example 1 to IL-5 according to Verification Example 1;
FIG. 5 is a graph showing the dose-response relationship of binding of Tab 1 and Tab2 to IL-5 in Verification Example 2;
FIG. 6 is a graph showing the dose-response relationship of IL-5-induced TF-1 cell proliferation neutralized with control antibody 1 (Tab1) and control antibody 2 (Tab2) in Verification Example 2;
FIG. 7 is a graph showing the dose-response relationship of IL-5-induced TF-1 proliferation neutralized with IL-5-specific Fc-fused single-domain antibody obtained in Example 3 in Verification Example 3; and
FIG. 8 is a graph showing the dose-response relationship of IL-5-induced TF-1 proliferation neutralized with different humanized Fc-fused single-domain antibodies obtained in Example 3 in Verification Example 3.

### DETAILED DESCRIPTION OF THE EMBODIMENTS

To make the objectives, technical solutions, and advantages of the embodiments of the present disclosure more comprehensible, the following clearly and completely describes the technical solutions in the embodiments of the present disclosure. Where no specific conditions are given in the examples, conventional conditions or conditions recommended by the manufacturer are followed. The reagents or instruments for which no manufacturers are noted are all common products commercially available from the market.

### Term definition

Herein, a "single-domain antibody" (sdAb), also known as a nanobody, is an antibody that naturally lacks light chains and including only one variable domain of heavy chain (VHH).

Herein, a "humanized antibody" refers to an antibody obtained by fusing the variable domain of heavy chain of a target antibody (such as an animal antibody) with the constant region of a human antibody, or an antibody obtained by grafting the complementarity-determining region (CDR 1-3 sequences) of a target antibody into the variable region of a human antibody, or an antibody obtained by subjecting a target antibody to amino acid mutation according to the characteristics of the framework region (FR1-4) of a human antibody. The humanized antibody may be obtained by synthesis or site-directed mutagenesis.

Herein, a "diabody" is a small bivalent and bispecific antibody fragment that can recognize two antigens simultaneously. Hollinger et al. linked the genes of the variable domain of light chain of the antibody of antigen A (VLA) to the variable domain of heavy chain of the antibody of antigen B (VHB) with short peptide molecules; and also linked VHA to VLB, and inserted two sets of chimeric genes into a bicistronic expression plasmid to construct a diabody expression plasmid. After expression, VLA-VHB was cross-linked to VHA-VLB to form a bispecific antibody. In the present disclosure, one of the antigens that can be recognized by the bivalent antibody is an IL-5 protein, and the other antigen that can be recognized by the bivalent antibody may be selected from any existing antigens.

Herein, a "multivalent antibody", also referred to as multi- antibody, refers to an antibody with its structure modified (similar to a diabody) that can recognize antibodies of various antigens concurrently (similar to a diabody). In the present disclosure, one of the antigens that can be recognized by the multivalent antibody is an IL-5 protein.

The "CDR" mentioned in this specification is a complementarity-determining region of an antibody. An antibody usually contains two variable regions: a variable domain of heavy chain and a variable domain of light chain. The variable domain of heavy chain or the variable domain of light chain usually includes three CDRs.

### Embodiments

An embodiment of the present disclosure provides an IL-5 binding molecule. The IL-5 binding molecule is capable of specifically binding to IL-5 and includes at least one of immunoglobulin single variable domains including complementarity determining regions CDR1, CDR2, and CDR3;

wherein an amino acid sequence of CDR1 is selected from any one of sequences as set forth in SEQ ID NOs. 43-49; an amino acid sequence of CDR2 is selected from any one of sequences as set forth in SEQ ID NOs. 50-56; and an amino acid sequence of CDR3 is selected from any one of sequences as set forth in SEQ ID NOs. 57-62.

Preferably, the amino acid sequences of the complementarity determining regions CDR1, CDR2, and CDR3 are as set forth in any one of (1)-(13):
(1) SEQ ID NOs. 43, 56, and 58;
(2) SEQ ID NOs. 49, 51, and 60;
(3) SEQ ID NOs. 47, 56, and 57;
(4) SEQ ID NOs. 45, 54, and 57;
(5) SEQ ID NOs. 46, 50, and 61;
(6) SEQ ID NOs. 47, 56, and 62;
(7) SEQ ID NOs. 48, 56, and 57;
(8) SEQ ID NOs. 45, 53, and 57;
(9) SEQ ID NOs. 44, 55, and 57;
(10) SEQ ID NOs. 43, 56, and 59;
(11) SEQ ID NOs. 43, 52, and 59;
(12) SEQ ID NOs. 47, 56, and 59; and
(13) SEQ ID NOs. 47, 52, and 59.

In an optional embodiment, the immunoglobulin single variable domains further include a framework region including FR1, FR2, FR3, and FR4. The structure of the single-domain antibody is: FR1-CDR1-FR2-CDR2-FR3-CDR3-FR4.

An amino acid sequence of FR1 is selected from any one of sequences as set forth in SEQ ID NOs. 63-68; an amino acid sequence of the FR2 is selected from any one of sequences as set forth in SEQ ID NOs. 69-72; an amino acid sequence of the FR3 is selected from any one of sequences as set forth in SEQ ID NOs. 73-86; and an amino acid sequence of FR4 is as set forth in SEQ ID No. 87.

Preferably, the framework regions FR1, FR2, and FR3 of the immunoglobulin single variable domains have sequences as set forth in any one of (14)-(28):
(14) SEQ ID NOs. 65, 72, and 85;
(15) SEQ ID NOs. 65, 72, and 82;
(16) SEQ ID NOs. 64, 71, and 86;
(17) SEQ ID NOs. 65, 72, and 73;
(18) SEQ ID NOs. 65, 72, and 84;
(19) SEQ ID NOs. 66, 69, and 83;
(20) SEQ ID NOs. 68, 72, and 76;
(21) SEQ ID NOs. 66, 69, and 79;
(22) SEQ ID NOs. 63, 72, and 85;
(23) SEQ ID NOs. 67, 70, and 80;
(24) SEQ ID NOs. 65, 72, and 78;
(25) SEQ ID NOs. 65, 72, and 77;
(26) SEQ ID NOs. 67, 70, and 81;
(27) SEQ ID NOs. 65, 72, and 74; and
(28) SEQ ID NOs. 65, 72, and 75.

Preferably, the immunoglobulin single variable domain is VHH.

Preferably, an amino acid sequence of the VHH is selected from any one of sequences as set forth in SEQ ID NOs. 1-12.

Preferably, the VHH is a humanized VHH.

Preferably, a sequence of the humanized VHH is selected from any one of sequences as set forth in SEQ ID NOs. 13-21.

Preferably, the binding molecule further includes an immunoglobulin Fc region linked to the VHH.

Preferably, the immunoglobulin Fc region is a human immunoglobulin Fc region.

Preferably, the human immunoglobulin Fc region is a human IgG4 Fc region.

An embodiment of the present disclosure provides an isolated nucleotide, encoding the IL-5 binding molecule according to any of the foregoing embodiments.

Preferably, a sequence of the nucleotide is selected from any one of sequences as set forth in SEQ ID NOs. 22-42.

An embodiment of the present disclosure provides a recombinant vector, including the isolated nucleotide according to any of the foregoing embodiments. In an optional embodiment, the recombinant vector may be a plasmid, a phage, or a viral vector.

An embodiment of the present disclosure provides a host cell, including the recombinant vector according to the foregoing embodiment. In an optional embodiment, the host cell may be a prokaryotic cell or a eukaryotic cell.

An embodiment of the present disclosure provides a preparation method of an IL-5 binding molecule, including culturing the host cell according to the foregoing embodiment to obtain the IL-5 binding molecule.

It is to be noted that, the binding molecule described in any of the above embodiments may be prepared by artificial synthesis, or may be obtained by first synthesizing its encoding gene and then carrying out biological expression.

An embodiment of the present disclosure provides a conjugate for binding an IL-5 protein, including a conjugation component and the IL-5 binding molecule according to any of the foregoing embodiments. The conjugation component is conjugated to the IL-5 binding molecule; and the conjugation component includes a marker and/or compound for detection.

Preferably, the marker for detection is a radioactive element.

An embodiment of the present disclosure provides a kit for detecting IL-5, including the IL-5 binding molecule according to any of the foregoing embodiments.

In addition, an embodiment of the present disclosure provides use of the IL-5 binding molecule in preparing IL-5-targeted drugs for treatment of a disease.

Preferably, the disease is selected from any one of asthma, allergic dermatitis, eczema, arthritis, herpes, chronic primary urticaria, scleroderma, hypertrophic scars, chronic obstructive pulmonary disease, atopic dermatitis, idiopathic pulmonary fibrosis, Kawasaki disease, sickle cell disease, Graves' disease, Sjögren's syndrome, autoimmune lymphoproliferative syndrome, autoimmune hemolytic anemia, Barrett's esophagus, autoimmune uveitis, tuberculosis, and kidney disease.

The features and performance of the present disclosure are further described in detail below with reference to examples.

### Example 1

Preparation of anti-IL-5 protein single-domain antibody.

### A. Construction of expression vector of human recombinant IL-5 protein:

The coding sequence of IL-5 was obtained by retrieval in NCBI, with its accession number of NM_000879.2. Encoding by the coding sequence provided an amino acid sequence with its accession number of NP_000870.1. The analysis about transmembrane regions and extracellular ends was carried out on the amino acid sequence corresponding to NP_000870.1 on TMHMM and SMART websites respectively. The analysis result showed that the IL-5 protein is a secretory protein without transmembrane region; and has 134 amino acids in its full length with a signal peptide at the amino acid Nos. 1-19. By means of gene synthesis, the nucleotide sequence encoding amino acid Nos. 20-134 of the IL-5 protein was cloned into the vector pcDNA3.4. The constructed vector was Sanger-sequenced and aligned with the original sequence. Upon successful verification, the recombinant plasmid was extracted in batches to remove endotoxin, and the suspension 293F cells were transfected for expression and purification of the target protein. The SDS-PAGE analysis results of the purified human recombinant IL-5 protein are shown in FIG. 1 (Marker: standard protein molecular weight gradient; hrIL-5-cHis: human recombinant IL-5 protein with a histidine tag at carboxyl terminal).

It can be learned from FIG. 1 that the purity of the expressed and purified human recombinant IL-5 protein is about 90%. The IL-5 protein obtained in this example is used for camel immunization and antibody screening.

### B. Construction of anti-IL-5 protein single-domain antibody library.

600 µg of purified human recombinant IL-5 protein obtained in step A was mixed with a Freund's complete adjuvant in an equal volume, to immunize an Inner Mongolia Alxa Bactrian camel, once a week, for a total of seven times. Except the first immunization, the other six immunizations were carried out by using a mixture of 300 µg of recombinant IL-5 protein and a Freund's incomplete adjuvant in an equal volume, to produce the anti-IL-5 antibody in the camel.

After the immunization, 100 mL of peripheral blood lymphocytes of the camel were drawn, and the RNA was extracted from the lymphocytes. The extracted total RNA was used to synthesize cDNA, and the heavy antibody variable region (VHH) was amplified through nested PCR with cDNA as a template. Then, the pMECS vector and the amplified VHH fragment were digested respectively by using restriction endonuclease, and the digested fragments and vectors were linked; the linked fragments were transformed into competent cells TG1, to construct a phage display library of the IL-5 protein, and the capacity of the library (recombinant TG1 cells) was determined to be about 1×10⁹.

### C. Screening of anti-IL-5 protein single-domain antibody.

200 µL of recombinant TG1 cells obtained in step B were inoculated in a 2×TY medium for incubation. During the incubation, 40 µL of helper phage VCSM13 was added to infect the TG1 cells, and cultured overnight to amplify the phage. The next day, the phage was precipitated with PEG/NaCl, and the amplified phage was collected by centrifugation, to obtain the amplified phage library.

500 µg of IL-5 protein diluted in NaHCOs (100 mM, pH 8.3) was conjugated in an ELISA plate at 4°C overnight. Negative control wells were also provided. On day 2, 200 µL of 3% skim milk was added for blocking at room temperature for 2 h. After the blocking, 100 µL of amplified phage library (about 2 x 10¹¹ phage particles) was added to act at room temperature for 1 h. After 1 hour of action, the plate was washed with PBS and 0.05% Tween-20 for five times to wash away unbound phages. The phage that specifically binds to the IL-5 protein was dissociated with trypsin in a final concentration of 2.5 mg/mL to infect E. coli TG1 cells in a logarithmic growth phase. The infected E. coli TG1 cells were cultured at 37°C for 1 h to produce and collect phages for next screening. The same screening process was repeated for another round to enable gradual enrichment. For fold enrichment of more than 10x, the enrichment effect is shown in FIG. 2 and Table 1.

**Table 1 Enrichment effect**

| | Round 1 | Round 2 | Round 3 |
|---|---|---|---|
| I/E | 8000 | 35.71428571 | 4 |
| P/N | 3.125 | 180.6451613 | 2500 |

It can be seen from the results that for the enrichment at round 3, PIN is 2500, and I/E is 4.

### D. Screening of specific positive clones against IL-5 by phage ELISA.

When the fold enrichment is more than 10, 400 single colonies were selected from the screened positive clones and inoculated into TB medium containing 100 µg/mL ampicillin in a 96-deep well plate. A blank control was also provided. The plate was cultured at 37°C to a logarithmic phase, and then IPTG was added in a final concentration of 1 mM to culture overnight at 28°C.

A crude antibody extract was obtained by osmotic burst. The human recombinant IL-5 protein was diluted into NaHCOs (100 mM, pH 8.3), and 100 µg of recombinant IL-5 protein was coated in an ELISA plate at 4°C overnight. 100 µL of obtained crude antibody extract was transferred to the ELISA plate into which the antigen (recombinant IL-5 protein) was added, and incubated at room temperature for 1 h. The unbound antibodies were washed away with PBST. 100 µL of mouse anti-HAtag antibody (Thermo Fisher) at 1:2000 dilution was added, and incubated at room temperature for 1 h. The unbound antibodies were washed away with PBST. 100 µL of anti-rabbit HRP conjugate (horseradish peroxidase-labeled goat anti-rabbit antibody, Thermo Fisher) in 1:20000 dilution was added, and incubated at room temperature for 1 h. The unbound antibodies were washed away with PBST. A horseradish peroxidase color development solution was added to react at 37°C for 15 min, a stopping solution was added, and the absorbance was measured at a wavelength of 450 nm in a microplate reader. When the optical density (OD) of sample wells is more than 5 times that of control wells, the sample wells are determined as positive clone wells. The bacteria in the positive clone wells were transferred into an LB medium containing 100 µg/µL ampicillin on a shaker for plasmid extraction and sequencing. The gene sequence of each clone was analyzed according to the sequence alignment software Vector NTI. The strains with the same CDR1, CDR2, and CDR3 sequences are regarded as the same clone, and the strains with different sequences are regarded as different clones, to finally obtain an IL-5 protein-specific single-domain antibody. The single-domain antibody has an amino acid sequence of FR1-CDR1-FR2-CDR2-FR3-CDR3-FR4, to form the entire VHH. The obtained single-domain antibody recombinant plasmid (positive plasmid, target sequence) can be expressed in a prokaryotic system to finally obtain a single-domain antibody protein.

### E. Purification and expression of IL-5 protein-specific single-domain antibody in host E. coli.

The positive plasmids (pMECS-VHH) of different clones obtained by sequencing analysis in step D were electroporated into E. coli HB2151, spread across an LB+amp+glucose culture plate (i.e. containing ampicillin and glucose) and incubated at 37°C overnight. A single colony was picked and inoculated in 5 mL of LB medium containing ampicillin, and incubated on a shaker at 37°C overnight. 1 mL of overnight culture were inoculated into 330 mL of TB medium, and incubated on a shaker at 37°C. The absorbance was measured at the wavelength of 600 nm using a spectrometer and recorded as OD600. When the OD600 value was measured in a range from 0.6 to 0.9, 1M IPTG was added to incubate on a shaker at 28°C overnight. The E. coli was collected by centrifugation. A crude antibody extract was obtained by osmotic burst. The antibody was purified through a Ni-column by affinity chromatography. The purified single-domain antibodies are shown in Table 2.

**Table 2 Information of single-domain antibodies**

| Number of Antibody | Name of Antibody | FR1 | CDR1 | FR2 | CDR2 | FR3 | CDR3 | FR4 | Full-length amino acid sequence | Corresponding nucleotide encoding sequence |
|---|---|---|---|---|---|---|---|---|---|---|
| 1 | 1B3 | 65 | 43 | 72 | 56 | 85 | 58 | 87 | 1 | 22 |
| 2 | 1B8 | 65 | 43 | 72 | 56 | 82 | 58 | | 2 | 23 |
| 3 | 1F4 | 64 | 49 | 71 | 51 | 86 | 60 | | 3 | 24 |
| 4 | 2B3 | 65 | 47 | 72 | 56 | 73 | 57 | | 4 | 25 |
| 5 | 2F7 | 65 | 43 | 72 | 56 | 84 | 58 | | 5 | 26 |
| 6 | 2G6 | 65 | 45 | 72 | 54 | 85 | 57 | | 6 | 27 |
| 7 | 3G12 | 66 | 46 | 69 | 50 | 83 | 61 | | 7 | 28 |
| 8 | 4B 12 | 68 | 47 | 72 | 56 | 76 | 62 | | 8 | 29 |
| 9 | 4C3 | 66 | 46 | 69 | 50 | 79 | 61 | | 9 | 30 |
| 10 | 4D5 | 65 | 48 | 72 | 56 | 73 | 57 | | 10 | 31 |
| 11 | 4G8 | 65 | 45 | 72 | 53 | 85 | 57 | | 11 | 32 |
| 12 | 4H2 | 63 | 44 | 72 | 55 | 85 | 57 | | 12 | 33 |
| 13 | 1B3-V1 | 67 | 43 | 70 | 56 | 80 | 59 | | 13 | 34 |
| 14 | 1B3-V2 | 65 | 43 | 72 | 52 | 78 | 59 | | 14 | 35 |
| 15 | 1B3-V3 | 65 | 43 | 72 | 52 | 85 | 59 | | 15 | 36 |
| 16 | 1B3-V4 | 65 | 43 | 72 | 52 | 77 | 59 | | 16 | 37 |
| 17 | 1B3-V5 | 65 | 43 | 72 | 56 | 78 | 59 | | 17 | 38 |
| 18 | 2B3-V1 | 67 | 47 | 70 | 56 | 81 | 59 | | 18 | 39 |
| 19 | 2B3-V2 | 65 | 47 | 72 | 52 | 74 | 59 | | 19 | 40 |
| 20 | 2B3-V3 | 65 | 47 | 72 | 52 | 73 | 59 | | 20 | 41 |
| 21 | 2B3-V4 | 65 | 47 | 72 | 52 | 75 | 59 | | 21 | 42 |

| | | | | | | | | | | |
|---|---|---|---|---|---|---|---|---|---|---|
| Note: In Table 2, the numbers corresponding to CDRs 1-3 and FRs 1-4 are their SEQ ID Nos. | | | | | | | | | | |

### Example 2

### Humanization of anti-IL-5 single-domain antibody.

The humanization was carried out by humanizing the amino acids on protein surface and grafting of universal framework for single-domain antibody humanization.

Humanizing the amino acids on protein surface was carried out by the following steps. Homology modeling was carried out for antibody strains 1B3 and 2B3 using the Discovery Studio, a modeling software, with NbBcII10 antibody (PDB number: 3DWT) having a homologous sequence as the reference, and the relative accessibility of the amino acid to solvent was calculated according to the protein three-dimensional structure.

Grafting of universal framework for the VHH humanization was carried out by the following specific steps. First, the universal framework h-NbBcII10FGLA (PDB Accession No. 3EAK) for VHH humanization was designed and provided according to sequence homology and based on the nanobody NbBcII10 (PDB Accession No. 3DWT), by humanizing the amino acids on protein surface with reference to the humanized antibody DP-47, and modifying some amino acids of VHH sequence framework-2 as FGLA. The CDRs of h-NbBcII10FGLA which is used directly as the framework was replaced with those of the antibody strains 1B3 and 2B3 respectively, to achieve antibody humanization.

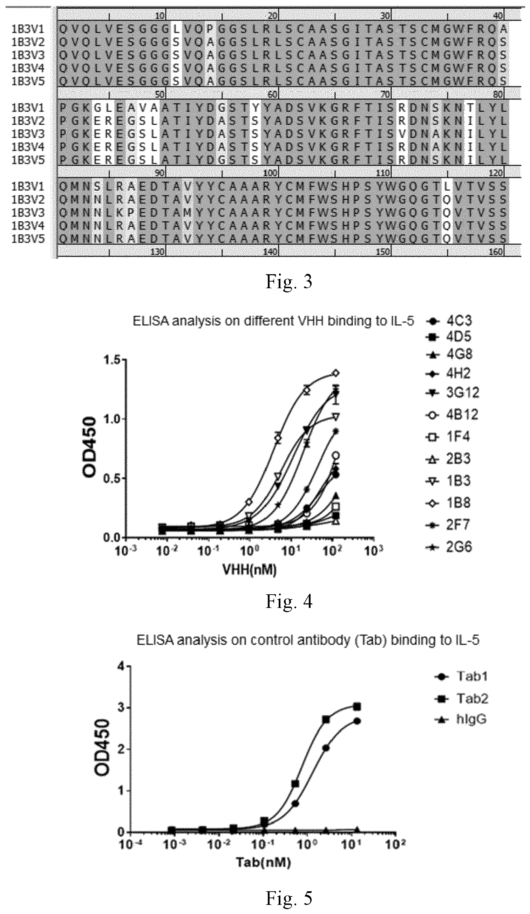

The antibody strains 1B3 and 2B3 were humanized to obtain five humanized antibody strain variants. Table 3 and Table 4 show the sequence numbers and amino acid changes of these humanized variants. The amino acid residues were numbered according to Kabat numbering system. "--J" in Table 3 and Table 4 indicates that there is mutation (substitution) at this position of the nanobody clone. Specifically, for example, "S11L" means that at position 11, S (serine) is replaced with L (leucine); and "S66T" means that at position 66, S (serine) is replaced with T (threonine). FIG. 3 shows the alignment results of the humanized sequences.

**Table 3 Sequence numbers of humanized variants of clones 1B3, 2B3 and amino acid changes in some variants**

| | S11L | A14P | S45A | E49G | R50L | G52A | S53V | L54A | S63A |
|---|---|---|---|---|---|---|---|---|---|
| 1B3-V1 | √ | √ | √ | √ | √ | √ | √ | √ | |
| 1B3-V2 | | | | | | | | | √ |
| 1B3-V3 | | | | | | | | | √ |
| 1B3-V4 | | | | | | | | | √ |
| 1B3-V5 | | | | | | | | | |
| 2B3-V1 | √ | √ | √ | √ | √ | √ | √ | √ | |
| 2B3-V2 | | | | | | | | | √ |
| 2B3-V3 | | | | | | | | | √ |
| 2B3-V4 | | | | | | | | | √ |

**Table 4 Sequence numbers of humanized variants of clone 1B3, 2B3 and amino acid changes in some variants**

| | S66T | V80R | A83S | I86T | L93S | K95R | P96A | M10IV | K108R |
|---|---|---|---|---|---|---|---|---|---|
| 1B3-V1 | √ | √ | √ | √ | √ | √ | √ | √ | √ |
| 1B3-V2 | | √ | √ | | | √ | √ | √ | √ |
| 1B3-V3 | | | | | | | | | √ |
| 1B3-V4 | | √ | | | | √ | √ | √ | √ |
| 1B3-V5 | | √ | √ | | | √ | √ | √ | √ |
| 2B3-V1 | √ | √ | √ | √ | √ | √ | √ | √ | √ |
| 2B3-V2 | | √ | √ | | | √ | √ | √ | √ |
| 2B3-V3 | | | | | | | | | √ |
| 2B3-V4 | | √ | | | | √ | √ | √ | √ |

### Example 3

### F. Construction of eukaryotic expression vector of Fc fusion protein of anti-IL-5 protein single-domain antibody.

The nucleotide sequence of the single-domain antibody screened in step D of Example 1 was obtained by Sanger sequencing. The above codon-optimized nucleotide sequence by means of sequence synthesis was inserted into the modified vector RJK-V4-hFC.

RJK-V4-hFC is a universal target vector for nanobodies, which is obtained by fusing Fc fragment of heavy-chain encoding sequence (NCBI Accession No.: AB776838.1) of human IgG with the commercial vector pCDNA3.4 (information about the vector are available on https://assets.thermofisher.com/TFS-Assets/LSG/manuals/pcdna3_4_topo_ta_cloning_kit_man.pdf) from Invitrogen. The vector contains the hinge CH2 and CH3 regions of the IgG heavy chain. The specific modification is as follows.

The XbaI and AgeI restriction enzyme cutting sites in pcDNA3.4 were selected. The multiple cloning site (MCS) and 6x His tag were introduced respectively at the 5' and 3' ends of the Fc fragment encoding sequence through overlap PCR. The above fragment was amplified by PCR using a pair of primers with XbaI and AgeI enzyme cutting sites respectively. The pcDNA3.4 and the amplified fragment with XbaI and AgeI enzyme cutting sites from primers were digested respectively using the restriction endonucleases XbaI and AgeI. The digested vector and the inserted fragment were linked using the T4 ligase, and then the linked product was transformed into E. coli, amplified, and verified by sequencing, to obtain a recombinant eukaryotic expression vector.

The constructed recombinant eukaryotic expression vector was transformed into DH5α E. coli, and incubated for plasmid maxiprep extraction, to remove endotoxin. The maxiprep extracted plasmid was sequenced for identification. The confirmed recombinant vector was used for subsequent transfection and expression in eukaryotic cells.

### G. Expression of Fc fusion protein of IL-5 protein-specific single-domain antibody in suspension ExpiCHO-S cells.

3 days before transfection, the ExpiCHO-S^{™} cells at a concentration of 2.5×10⁵/mL were passaged and expanded. The cells in a required volume by calculation were transferred into a 500 mL shake flask containing 120 mL (final volume) of ExpiCHO^{™} expression medium. The cells were incubated to a concentration of about 4×10⁶ to 6×10⁶ living cells/mL. One day before transfection, the ExpiCHO-S^{™} cells were diluted to 3.5×10⁶ living cells/mL, and cultured overnight. On the day of transfection, the cell density and living cell percentage were determined. Before the transfection, the cell density should reach about 7×10⁶ to 10×10⁶ living cells/mL. The cells were diluted to 6×10⁶ living cells/mL with a fresh ExpiCHO^{™} expression medium pre-warmed to 37°C. The cells in a required volume by calculation were transferred into a 500 mL shake flask containing 100 mL (final volume) of fresh pre-warmed ExpiCHO^{™} expression medium. The ExpiFectamine^{™}CHO reagent was mixed uniformly by gently inverting it upside down, and diluted with 3.7 mL of OptiPRO^{™} medium, shaken or mixed uniformly. The plasmid DNA (obtained in step F) was diluted with 4 mL of cooled OptiPRO^{™} medium, shaken and mixed uniformly. The ExpiFectamine CHO/plasmid DNA mixture was incubated at room temperature for 3 min, and then gently added into a prepared cell suspension during which the shake flask was gently shaken. The cells were shake-cultured in humidified air containing 8% CO₂ at 37°C. On day 1 after the transfection (18-22 hours later), 600 µL of ExpiFectamine^{™}CHO enhancer and 24 mL of ExpiCHO feed were added. About 8 days after the transfection (the cell viability was less than 70%), the supernatant was collected.

### H. Expression of Fc fusion protein of anti-IL-5 protein single-domain antibody in suspension 293F cells.

3 days before transfection, the 293F cells at a concentration of 2.5×10⁵/mL were passaged and expanded. The cells in a required volume by calculation were transferred into a 500 mL shake flask containing 120 mL (final volume) of fresh pre-warmed OPM-293 CD05 medium. The cells were incubated to a concentration of about 2×10⁶ to 3×10⁶ living cells/mL. On the day of transfection, the cell density and living cell percentage were determined. Before the transfection, the cell density should reach about 2×10⁶ to 3×10⁶ living cells/mL. The cells were diluted to 1×10⁶ living cells/mL with a pre-warmed OPM-293 CD05 medium. The cells in a required volume by calculation were transferred into a 500 mL shake flask containing 100 mL (final volume) of fresh pre-warmed medium. The PEI (1 mg/mL) reagent was diluted with 4 mL of Opti-MEM medium, and shaken or mixed uniformly by pipetting. The plasmid DNA (obtained in step F) was diluted with 4 mL of Opt-MEM medium, shaken and mixed uniformly, filtered with a 0.22 µm filter head, and incubated at room temperature for 5 min. The diluted PEI reagent was added in the diluted DNA, and inverted upside down to mix uniformly. The PEI/plasmid DNA mixture was incubated at room temperature for 15-20 min, and then gently added in a prepared cell suspension during which the shake flask was gently shaken. The cells were shake-cultured at 37°C in 5% CO₂ at 120 rpm. At 24 h and 72 h after the transfection, 5 mL of OPM-CHO PFF05 feed was added. About 7 days after the transfection (the cell viability was less than 70%), the supernatant was collected.

### F. Purification of Fc fusion protein of anti-IL-5 protein single-domain antibody.

The expression supernatant of the Fc fusion protein obtained in step G or H was filtered with a 0.45 µm disposable filter head to remove insoluble impurities. The filtrate was purified by affinity chromatography using a protein purifier, with an agarose filler conjugated to Protein A based on the binding of human Fc fusion protein to Protein A. The filtrate was allowed to pass through a Protein A prepacked column at a flow rate of 1 mL/min. In this step, the target protein in the filtrate binds to the filler. The impurity proteins binding to the column were washed away with low-salt and high-salt buffers. The target protein binding to the column was eluted with a low-pH buffer. The eluted solution was quickly added in a Tris-HCl solution at pH 9.0 for neutralization.

The neutralized protein solution was dialyzed and then subjected to SDS-PAGE analysis. The protein having a purity of 95% or more and a concentration of 0.5 mg/mL or more was cryopreserved and ready for use.

### Verification Example 1

### Determination of dose-response relationship of binding of IL-5 protein-specific single-domain antibody provided in Example 1.

50 µL of 1 µg/mL IL-5 protein was coated on an ELISA plate at 4°C overnight. The plate was washed. 200 µL of 5% milk was added for blocking at 37°C for 1 h. The IL-5 protein-specific single-domain antibody (VHH) obtained in Example 1 was diluted to 2 µg/mL, and then diluted by a 5-fold gradient, in a total of 8 concentration gradients. The plate was washed. 50 µL of antibody was added in duplicate wells, and then incubated at 37°C for 1 h. The plate was washed. 50 µL of mouse anti-HA tag HRP secondary antibody was added, and then incubated at 37°C for 30 min. The plate was washed (for multiple times). 50 µL of TMB that had been restored to room temperature was added to react at room temperature in the dark for 15 min. 50 µL of stopping solution (1N HCl) was added. The resultant was measured using a microplate reader and then recorded. The curve was plotted. The concentration for 50% of maximal effect (EC50), which is the concentration that elicits 50% of the maximal effect, was calculated and shown in FIG. 4 and Table 5.

**Table 5 EC50 results**

| | 4C3 | 4D5 | 4G8 | 3G12 | 4H2 | 4B 12 | 1F4 | 2B3 | 1B3 | 1B8 | 2F7 | 2G6 |
|---|---|---|---|---|---|---|---|---|---|---|---|---|
| EC50 | 53.02 | 88.67 | ∼479939 | 11.23 | 48.19 | ∼175524 | ∼21637 | ∼3445962 | 5.582 | 3.76 | 44.83 | 19.84 |

### Verification Example 2

### Expression and purification of Tool antibody (Tab) targeting human IL-5 protein.

Tab1 (control antibody 1) and Tab2 (control antibody 2) were obtained. Tab1 is mepolizumab having a sequence as set forth in patent US7982005B2. Tab2 is reslizumab having a sequence as set forth in patent US6056957.

The searched Tab1 and Tab2 sequences were entrusted to General Biol (Anhui) Co., Ltd. for codon optimization for mammalian cell expression system, and cloned into the pcDNA3.1 vector respectively.

The plasmid-positive bacteria screened out for resistance were amplified, and plasmids were extracted by using a plasmid midiprep kit (Macherey Nagel, Cat#740412.50). 100 µg of plasmids (50 µg of heavy chains and 50 µg of light chains) per 100 mL of cells were added, and transiently transfected and expressed in 293F cells (FreeStyle 293 Expression medium, Thermo, Cat#12338026+F-68, Thermo, Cat#24040032) with PEI. 6-24 h after the transfection, 5% volume of 10% Peptone (Sigma, Cat#P0521-100G) was added and cultured in 8% CO₂ at 130 rpm for about 7-8 days. When the cell viability dropped to 50%, the expression supernatant was collected and purified through a Protein A (GE, Cat#17-5438-02) gravity column. After PBS dialysis, the concentration was determined by Nanodrop, the purify was identified by SEC, and the binding was verified by indirect ELISA. The Tab antibody obtained by this method has a concentration of not less than 2 mg/ml and a purity of more than 94%. The EC50 of binding to IL-5 protein (Novoprotein, Cat#CS 33) are shown in FIG. 5 and Table 6.

**Table 6 EC50 results**

| | Tab1 | Tab2 | HlgG |
|---|---|---|---|
| EC50 | 1.321 | 0.7574 | -4.591 |

### Experiment of TF1 cell proliferation induced by human recombinant IL-5 protein and neutralized by Tool antibody (Tab).

Experiment of TF 1 cell proliferation induced by human recombinant IL-5 protein: The TF-1 cells passaged for 3-4 passages after resuscitation were inoculated in a 96-well plate in 10000 cells/well. The human IL-5 protein was formulated into a solution with a maximum concentration of 500 ng/mL, and diluted by a 5-fold gradient. The gradient-diluted IL-5 protein solution was added in the cell culture wells in an equal volume to the cell culture medium. After incubation for 72 h, the cell viability was detected with a luminescent cell viability assay kit. The EC80 of IL-5-induced TF-1 cell proliferation was calculated according to the detection results. EC80 (concentration for 80% of maximal effect) is the concentration that elicits 80% of the maximal effect.

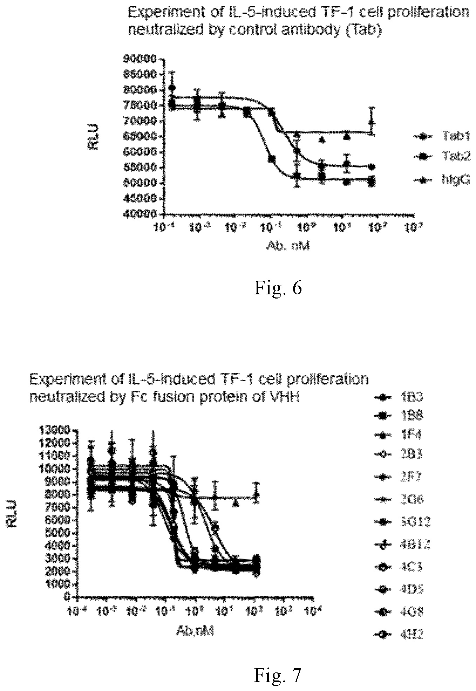

Experiment of human IL-5-induced TF1 cell proliferation neutralized by Tab: The TF-1 cells passaged for 3-4 passages after resuscitation were inoculated in a 96-well plate in 10000 cells/well. Tab1 and Tab2 were formulated into a 10 µg/mL solution, and diluted by a 5-fold gradient. The gradient-diluted Tab was mixed with IL-5 at a concentration of EC80 obtained in the proliferation experiment in 1:1 to prepare a mixed solution. The mixed solution was added in the cell culture wells in an equal volume to the cell culture medium. After incubation for 72 h, the cell viability was detected with a luminescent cell viability assay kit. The EC50 of Tab1 and Tab2 for neutralizing IL-5-induced TF-1 cell proliferation was calculated according to the detection results and shown in FIG. 6 and Table 7.

**Table 7 EC50 results**

| | Tab1 | Tab2 | HlgG |
|---|---|---|---|
| EC50 | 0.2338 | 0.06639 | ∼0.1323 |

### Detection of IL-5-induced TF1 cell proliferation neutralized by Fc fusion protein of anti-IL-5 protein single-domain antibody.

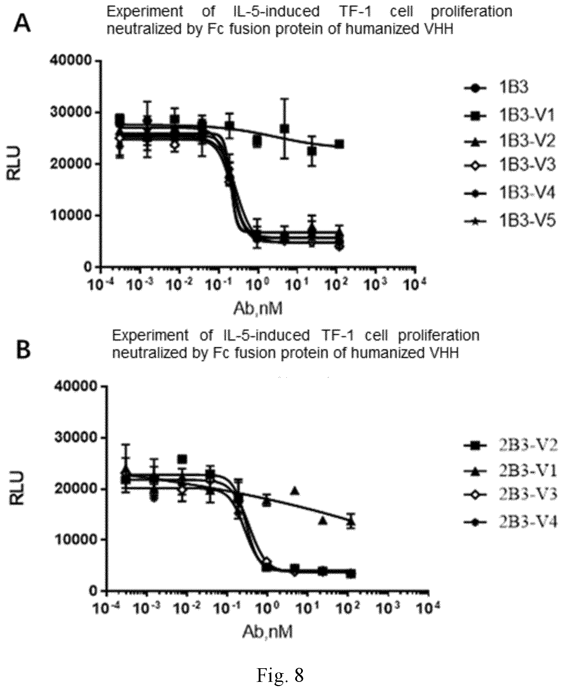

The TF-1 cells passaged for 3-4 passages after resuscitation were inoculated in a 96-well plate in 10000 cells/well. Tab1, Tab2, and the Fc fusion protein of the single-domain antibody provided in Example 3-I were formulated into a 10 µg/mL solution, and diluted by a 5-fold gradient. The gradient-diluted Tab and single-domain antibody were mixed respectively with IL-5 protein at a concentration of EC80 obtained in the proliferation experiment in 1:1 to prepare a mixed solution. The mixed solution was added in the cell culture wells in an equal volume to the cell culture medium. After incubation for 72 h, the cell viability was detected with a luminescent cell viability assay kit. The EC50 of different single-domain antibodies for neutralizing IL-5-induced TF-1 cell proliferation was calculated according to the detection results and shown in FIGs. 7-8 and Tables 8-9.

**Table 8 EC50 results**

| | 1B3 | 1B8 | 1F4 | 2B3 | 2F7 | 2G6 | 3G12 | 4B 12 | 4C3 | 4D5 | 4G8 | 4H2 |
|---|---|---|---|---|---|---|---|---|---|---|---|---|
| EC50 | 0.09556 | 0.1752 | 0.09999 | 0.1557 | 0.1496 | ∼0.1881 | 2.089 | ∼0.1961 | 4.397 | 0.4275 | ∼0.1757 | ∼0.1894 |

**Table 9 EC50 results**

| | 1B3 | 1B3-V1 | 1B3-V2 | 1B3-V3 | 1B3-V4 | 1B3-V5 | 2B3-V1 | 2B3-V2 | 2B3-V3 | 2B3-V4 |
|---|---|---|---|---|---|---|---|---|---|---|
| EC50 | 0.2019 | 2.817 | 0.2021 | 0.2192 | 0.2374 | 0.2743 | ∼6.737e+029 | 0.2899 | 0.3487 | 0.2701 |

### Affinity kinetics detection of IL-5 binding molecule

Formulation of SD buffer: A proper amount of bovine serum albumin and Tween 20 were dissolved in 1 x PBS (pH 7.4), so that the mass (or volume) fractions of bovine serum albumin and Tween 20 were 0.1% and 0.02% respectively. The IL-5 binding molecule was formulated in the SD buffer to a concentration of 10 µg/mL. Formulation of antigen working solution: the antigen was formulated in the SD buffer to 200 nM, and then diluted by a 2-fold gradient in a total of 5 concentration gradients. In addition, a blank control of SD buffer was also provided. A proper amount of 0.1M glycine stock solution was diluted by 10-fold in deionized water and mixed uniformly, to obtain a regeneration solution. Octet 96 and the Data Acquisition software in its supporting computer were run. The bottom and side of the acquisition probe were cleaned using a lens tissue with a proper amount of 75% ethanol. The instrument was pre-warmed for 15 min or more. Sensor pre-wetting: sensor was soaked in the SD buffer for 10 min or more before the assay. Then, the machine procedure was set according to baseline→antibody→baseline→binding antigen→dissociating antigen→regenerating sensor for assay operation.

The affinity kinetics parameters for the IL-5 binding molecule tested specifically are shown in Table 10.

**Table 10 Affinity kinetics results of clones 1B3 and 2B3, and their humanized variants binding to IL-5**

| Clone name | KD (M) | KD Error | Ka (l/Ms) | Ka Error | Kd (1/s) | Kd Error | X² | R² |
|---|---|---|---|---|---|---|---|---|
| Tab 1 | 0.99E-09 | 6.49E-11 | 8.26E+04 | 4.50E+02 | 8.22E-05 | 5.34E-06 | 0.1917 | 0.9978 |
| 2B3-V2 | 2.06E-09 | 4.60E-11 | 1.97E+05 | 1.71E+03 | 4.06E-04 | 8.35E-06 | 0.102 | 0.9838 |
| 2B3 | 1.60E-09 | 4.03E-11 | 1.84E+05 | 1.35E+03 | 2.95E-04 | 7.11E-06 | 0.1618 | 0.9892 |
| 1B3-V2 | 1.02E-09 | 2.78E-11 | 1.80E+05 | 9.48E+02 | 1.84E-04 | 4.91E-06 | 0.0988 | 0.9941 |
| 1B3 | 0.88E-09 | 3.38E-11 | 1.49E+05 | 7.81E+02 | 1.32E-04 | 5.00E-06 | 0.1979 | 0.9954 |

| | | | | | | | | |
|---|---|---|---|---|---|---|---|---|
| KD: Affinity constant in moles (M). Ka: Association rate constant in the reciprocal of molar time (1/Ms). Kd: Dissociation rate constant in the reciprocal of time. R²: Degree of fitting, that is, the degree of fitting between a measured curve and a fitted curve. R² closer to 1 indicates that the fitted value is closer to the measured value, and in this system, R² should be at least >0.95. X²: the statistical parameter performance of the values measured by the system, which should be <3, and a smaller measured value is more reliable. | | | | | | | | |

Other error values are the error values of the corresponding parameters, which should be more than an order of magnitude (10-fold) smaller than the corresponding parameters or less.

The results showed that, compared with the tool antibody, the clone 1B3 has a slightly small affinity kinetic constant, and other clones have a slightly large affinity kinetic constant, but there is no significant difference in the affinity kinetic constant among the tested samples, and there is no statistical difference.

The foregoing descriptions are merely preferred examples of the present disclosure and are not intended to limit the present disclosure. A person skilled in the art may make various alterations and variations to the present disclosure. Any modification, equivalent replacement, or improvement made within the spirit and principle of the present disclosure shall fall within the protection scope of the present disclosure.

## Claims

1. An IL-5 binding molecule, being capable of specifically binding to IL-5 and comprising at least one of immunoglobulin single variable domains comprising complementarity determining regions CDR1, CDR2, and CDR3,
wherein CDR1 has an amino acid sequence selected from any one of sequences as set forth in SEQ ID NOs. 43-49; CDR2 has an amino acid sequence selected from any one of sequences as set forth in SEQ ID NOs. 50-56; and CDR3 has an amino acid sequence selected from any one of sequences as set forth in SEQ ID NOs. 57-62.

2. The IL-5 binding molecule according to claim 1, wherein the amino acid sequences of the complementarity determining regions CDR1, CDR2, and CDR3 are as set forth in any one of (1)-(13):
(1) SEQ ID NOs. 43, 56, and 58;
(2) SEQ ID NOs. 49, 51, and 60;
(3) SEQ ID NOs. 47, 56, and 57;
(4) SEQ ID NOs. 45, 54, and 57;
(5) SEQ ID NOs. 46, 50, and 61;
(6) SEQ ID NOs. 47, 56, and 62;
(7) SEQ ID NOs. 48, 56, and 57;
(8) SEQ ID NOs. 45, 53, and 57;
(9) SEQ ID NOs. 44, 55, and 57;
(10) SEQ ID NOs. 43, 56, and 59;
(11) SEQ ID NOs. 43, 52, and 59;
(12) SEQ ID NOs. 47, 56, and 59; and
(13) SEQ ID NOs. 47, 52, and 59;
preferably, the immunoglobulin single variable domain further comprises a framework region comprising FR1, FR2, FR3, and FR4,
wherein FR1 has an amino acid sequence selected from any one of sequences as set forth in SEQ ID NOs. 63-68; FR2 has an amino acid sequence selected from any one of sequences as set forth in SEQ ID NOs. 69-72; FR3 has an amino acid sequence selected from any one of sequences as set forth in SEQ ID NOs. 73-86; and FR4 has an amino acid sequence as set forth in SEQ ID NO. 87; and
preferably, the framework regions FR1, FR2, and FR3 of the immunoglobulin single variable domain have sequences as set forth in any one of (14)-(28):
(14) SEQ ID NOs. 65, 72, and 85;
(15) SEQ ID NOs. 65, 72, and 82;
(16) SEQ ID NOs. 64, 71, and 86;
(17) SEQ ID NOs. 65, 72, and 73;
(18) SEQ ID NOs. 65, 72, and 84;
(19) SEQ ID NOs. 66, 69, and 83;
(20) SEQ ID NOs. 68, 72, and 76;
(21) SEQ ID NOs. 66, 69, and 79;
(22) SEQ ID NOs. 63, 72, and 85;
(23) SEQ ID NOs. 67, 70, and 80;
(24) SEQ ID NOs. 65, 72, and 78;
(25) SEQ ID NOs. 65, 72, and 77;
(26) SEQ ID NOs. 67, 70, and 81;
(27) SEQ ID NOs. 65, 72, and 74; and
(28) SEQ ID NOs. 65, 72, and 75.

3. The IL-5 binding molecule according to claim 2, wherein the immunoglobulin single variable domain is VHH;
preferably, the VHH has an amino acid sequence selected from any one of sequences as set forth in SEQ ID NOs. 1-12;
preferably, the VHH is a humanized VHH; and
preferably, the humanized VHH has a sequence selected from any one of sequences as set forth in SEQ ID NOs. 13-21.

4. The IL-5 binding molecule according to any one of claims 1 to 3, further comprising an immunoglobulin Fc region linked to the VHH.

5. The IL-5 binding molecule according to claim 4, wherein the immunoglobulin Fc region is a human immunoglobulin Fc region;
preferably, the human immunoglobulin Fc region is a human IgG4 Fc region.

6. An isolated nucleotide encoding the IL-5 binding molecule according to any one of claims 1 to 5;
preferably, the nucleotide has a sequence selected from any one of sequences as set forth in SEQ ID NOs. 22-42.

7. A recombinant vector comprising the isolated nucleotide according to claim 6.

8. A host cell comprising the recombinant vector according to claim 7.

9. A method for preparing an IL-5 binding molecule, comprising culturing the host cell according to claim 8 to obtain the IL-5 binding molecule.

10. A conjugate for binding to an IL-5 protein, comprising a conjugation component and the IL-5 binding molecule according to any one of claims 1 to 5, wherein the conjugation component is conjugated to the binding molecule; and the conjugation component comprises a marker and/or compound for detection;
preferably, the marker for detection is a radioactive element.

11. A kit for detecting an IL-5 protein, comprising the IL-5 binding molecule according to any one of claims 1 to 5.

12. Use of the IL-5 binding molecule according to any one of claims 1 to 5 in preparing IL-5-targeted drugs for treatment of a disease;
preferably, the disease is selected from any one of asthma, allergic dermatitis, eczema, arthritis, herpes, chronic primary urticaria, scleroderma, hypertrophic scars, chronic obstructive pulmonary disease, atopic dermatitis, idiopathic pulmonary fibrosis, Kawasaki disease, sickle cell disease, Graves' disease, Sjögren's syndrome, autoimmune lymphoproliferative syndrome, autoimmune hemolytic anemia, Barrett's esophagus, autoimmune uveitis, tuberculosis, and kidney disease.
